# EUROPEAN PATENT APPLICATION

(11) **EP 1 632 247 A1**
(43) Date of publication of application: **08.03.2006**
(21) Application number: 04020994.2
(22) Date of filing: 03.09.2004
(51) Int. Cl.: A61K 39/135

(54) **Nucleic acid sequences encoding FMDV proteins capable of associating into a virus-like particle**

(71) Applicant: CONSEJO SUPERIOR DE INVESTIGACIONES CIENTIFICAS, 28006 Madrid (ES)
(72) Inventor: Enjuanes Sanches, Luis, La Moraleja, ES-28109 Madrid (ES); Domingo Solans, Esteban, Dr., 28770 Colmenar Viejo, Madrid (ES)
(74) Representative: UEXKÜLL & STOLBERG

(57) **Abstract**

The present invention relates to nucleic acids comprising:
(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and
(b) sequences encoding one or more proteins of foot and mouth disease virus (FMDV), wherein the one or more proteins are capable of associating into a virus-like particle (VLP) that does not contain any infectious nucleic acid.

The present invention further relates to vectors, virus particles and host cells comprising these nucleic acids as well as their use for the preparation of pharmaceutical compositions, specifically for the preparation of vaccines.

## Description

The present invention is directed to nucleic acids comprising sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell. The nucleic acids of the present invention further encode one or more proteins of a foot and mouth disease virus (FMDV), wherein these one or more proteins are capable of associating into a virus-like particle (VLP) that does not contain any infectious nucleic acid. The present invention is further directed to the use of these nucleic acids for the preparation of pharmaceutical compositions in general and specifically for the preparation of vaccines.

### TECHNICAL BACKGROUND

Therapy approaches that involve the insertion of a functional gene into a cell to achieve a therapeutic effect are also referred to as gene therapy approaches, as the gene serves as a drug. Gene therapy is a technique primarily for correcting defective genes responsible for disease development.

A carrier molecule also referred to as a vector is used to deliver the therapeutic gene to the patient's target cells. Currently, the most common vector is a virus that has been genetically altered to carry human or animal genes. Viruses have evolved a way of encapsulating and delivering their genes to human or animal cells in a pathogenic manner. Scientists have taken advantage of this capability and manipulate the virus genome to remove disease-causing genes and insert therapeutic genes.

Target cells such as the patient's liver or lung cells are infected with the viral vector. The vector then unloads its genetic material containing the therapeutic gene into the target cell. The generation of a functional protein product from the therapeutic gene restores the target cell to a normal state.

In an alternative approach, these viral vectors were used for expressing heterologous genes that cause an immunogenic response in the subject receiving the vector and thus immunize that subject. In that case the viral vector serves as a vaccine.

Transmissible gastroenteritis virus is a member of the family of coronaviruses. Coronaviruses are ssRNA(+) viruses which have the largest genome so far found in RNA viruses with a length between 25 and 31 kilobases (kb; see Siddell S.G. 1995, The Coronaviridae). When a coronavirus infects a cell, the genomic RNA (gRNA) replicates in the cytoplasm and a set of subgenomic RNAs (sgRNA) of positive and negative polarity is produced (Sethna et al., 1989; Sawicki & Sawicki, J.Virol., 1990; and Van der Most and Spaan, The Coronaviridae).

Due to the fact that the coronaviruses replicate in the cytoplasm, use of coronaviruses as a vector for gene therapy and vaccination has been suggested. Specifically, defective interfering (DI) genomes of coronaviruses were produced. These DI genomes are deletion mutants which require the presence of a complementing or helper virus for replication and/or transcription (see Chang et al., 1994; WO97/34008; Spanish patent application P9600620; Izeta et al., 1999; and Sánchez et al., 1999).

A respective system was used in the art to generate immune responses in an animal which received a composition containing a DI genome which amongst others contained sequences encoding a heterologous reporter gene or a gene derived from a different infectious agent (porcine reproductive and respiratory disease virus, PRRSV; see Alonso et al., 2002a, 2002b).

The entire genome of a coronavirus was cloned in the form of an infectious cDNA (Almazan et al., 2000 and WO01/39797). The cloning of the entire genome allowed the preparation of infectious vectors containing heterologous sequences suitable for expression of large proteins in a host cell. In the examples of WO01/39797 sequences encoding the ORF 5 of the porcine reproductive and respiratory disease virus (PRRSV) were expressed in a viral vector derived from a coronavirus.

The potential of the cloned viral genome for expression of heterologous sequences was further reviewed in Enjuanes et al., 2003.

Using the cloned virus the structure of the genome and relevance of the coronaviral genes for infection were assessed by preparing deletion mutants. It was found that genes 3a, 3b and 7 are non-essential for replication of the viral nucleic acid and that absence of the genes reduces pathogenicity of the virus (Ortego et al., 2002 and 2003; Sola et al., 2003).

The preparation of a vaccine requires that the viral sequences are expressed in a manner which closely resembles the virus particle during infection. Therefore it was suggested to prepare virus-like particles, i.e. an association of several proteins of one virus that resembles the virus. The virus-like particles (VLPs) were administered as a vaccine for example intranasally (see Schwartz-Cornil et al., 2002). Kim et al. (2002), for example describe the production of rotavirus virus-like particles consisting of bovine VP6 and VP2 proteins by expressing the genes encoding these proteins in recombinant baculoviruses.

All of the above approaches are directed to the use of viral proteins as a vaccine. In a different approach WO02/092827 suggested a vaccine comprising coronavirus virus-like particles from a coronavirus vector backbone. Large parts of the coronaviral genome had been (at least functionally) deleted. However, in that publication virus-like particles were defined as particles containing proteins and nucleic acid (viral RNA). The virus like particle thus contains a potentially replicative nucleic acid and is thus much less safe than the VLPs administered directly as a protein vaccine.

The problem underlying the present invention thus resides in providing vaccine vectors with good safety and immunogenicity.

### SUMMARY OF THE INVENTION

According to a first aspect of the present invention a nucleic acid is provided which comprises:
(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and
(b) sequences encoding one or more proteins of foot and mouth disease virus (FMDV), wherein these proteins are capable of associating into a virus-like particle (VLP) that does not contain any infectious nucleic acid.

The replication competent TGEV sequences need not but may further encode other TGEV proteins. The TGEV sequences may thus encode a fully infectious TGEV virus and the sequences of FMDV or just comprise a replication competent nucleic acid.

The present invention further relates to vectors comprising a respective nucleic acid and host cells comprising the vector. The host cells may be capable of complementing TGEV genes that may have been deleted from the nucleic acids of the present invention. The host cell thus may be a packaging cell line or may contain a helper virus expressing TGEV genes, so that a TGEV virus particle is formed that comprises the sequences of FMDV encoding proteins capable of associating into a virus-like particle (VLP) that does not contain any infectious nucleic acid. Virus particles obtained by association of the TGEV coat proteins with the nucleic acids of the present invention are an especially preferred embodiment of the present invention (corresponding virus particles have also been referred to as pseudoviruses).

Finally, the present invention is also directed to the medical use of the nucleic acids, the virus vectors, the host cells and the virus particles, specifically to the use as a vaccine for treating or protecting mammals, such as a ruminant or a swine against infectious diseases. The vaccine can thus be administered to an animal to reduce or eliminate the symptoms of a subsequent infection of a wild-type virus.

### DETAILED DESCRIPTION OF THE INVENTION

The present invention is thus directed to a nucleic acid comprising:
(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and
(b) sequences encoding one or more proteins of foot and mouth disease virus (FMDV), wherein these one or more proteins are capable of associating into a virus-like particle (VLP) that does not contain any infectious nucleic acid.

The present inventors have surprisingly found that expression of heterologous proteins in the context of the TGEV vector backbone allows the foreign proteins to associate into a virus-like particle, when the nucleic acid is expressed in a host cell.

In accordance with the present invention an association of viral proteins is referred to as a "virus-like particle" if it comprises a covalently coupled or otherwise linked association of one viral protein with at least a part of a further viral protein that may have the same or a different sequence. Preferably the particle comprises an association of several or all of the viral coat proteins. The "virus-like particle" does not contain any replicating nucleic acid and is by itself thus not capable of causing an infection.

In other words, the nucleic acids of the present invention allow expression and secretion of virus-like particles, which will initiate immune responses closely mimicking the immune response caused by the wild-type virus particle. At the same time the virus-like particles do not contain any infectious nucleic acids, i.e. are extremely safe and cannot per se cause any infection.

Although the VLPs thus do not contain a nucleic acid, the proteins of the VLPs are encoded by a nucleic acid of the present invention. In its broadest aspect that nucleic acid is further characterized as a nucleic acid encoding replication competent TGEV sequences that means sequences encoding a TGEV replicase under the control of expression regulatory sequences so that expression of the replicase in a cell containing the nucleic acid will initiate replication of the nucleic acid and thus increase the number of nucleic acids in the cell. Once a cell is infected by the nucleic acids of the present invention, the gene for the replicase will be expressed and the nucleic acid will be replicated. The more copies of the nucleic acid are present in the cell the more VLPs will be expressed.

The replication competent TGEV vector may be infectious or not. A nucleic acid that contains at least all sequences necessary for replication of the nucleic acid, produces one or several coat proteins and associates with the coat proteins to a viral particle that will allow infection of other cells is referred to as an infectious nucleic acid in accordance with the present invention.

The non-infectious vector is more safe, as it does not contain sequences encoding a protein capable of associating with the nucleic acid to form an infectious viral particle.

However, the infectious TGEV vector also has advantages, as it will spread in the vaccinated animal and thus increase the immune response against the VLPs. According to one aspect of the present invention, the TGEV vector encodes different coat proteins that will allow infection of different host cells of the same organism.

In an especially preferred aspect, the present invention provides a virus particle that comprises the above nucleic acid and at least one TGEV coat protein. The virus particle may comprise more than one and even all TGEV coat proteins. A corresponding virus particle will be capable of entering a host cell by way of infection. However, the nucleic acid of such a virus particle may still be infectious or non-infectious, as it need not encode all of the TGEV coat proteins necessary to produce a virus particle. If the nucleic acid is a non-infectious nucleic acid in the sense of the present application, the virus particle is prepared using a packaging host cell or a helper virus that complements the TGEV genes. The use of packaging host cells or helper viruses for obtaining virus particles comprising an incomplete genome of a virus is well known in the art. This way of proceeding has specific advantages, as the virus particle is per se infectious (i.e. can infect a cell once) but the nucleic acid is not capable of producing further infectious virus particles. In other words, neither the sequences derived from TGEV nor the sequences derived from FMDV encode proteins that will be capable of associating with the nucleic acid to form a new virus particle. These virus particles thus are extremely safe and still provide a high immunogenic response against the VLPs expressed by the nucleic acids.

According to an alternative embodiment of the present invention the TGEV infectious viral particles obtainable from the association of TGEV proteins and the nucleic acid sequences are attenuated viral particles. This has the advantage that the subject to be treated using the nucleic acids of the present invention will be vaccinated at the same time against TGEV and against FMDV.

The nucleic acids of the present invention may comprise sequences encoding all proteins of TGEV. Alternatively, the nucleic acids may comprise sequences only encoding the TGEV proteins needed for a replication competent TGEV vector. The nucleic acid thus preferably encodes the TGEV replicase. According to an especially preferred embodiment, the nucleic acid encodes a replication competent but non-infectious TGEV vector that comprises sequences encoding the TGEV replicase and the TGEV N protein and none of the other TGEV proteins. This vector has the specific advantage that the TGEV vector will be highly amplified in the host cell and thus produce large amounts of the VLPs. At the same time this vector is extremely safe as it is non-infectious.

The term "nucleic acids encoding TGEV proteins" is used herein to refer to nucleic acid sequences as disclosed in Penzes et al., 2001 or nucleic acid sequences having a homology of at least 60%, preferably at least 75% and most preferably at least 95% to these sequences. For example specific alternative sequences may be used to differentiate between TGEV vaccinated animals and TGEV infected animals (as outlined in more detail below). In the TGEV based vector exemplified in the present application corresponding nucleotide substitutions have been introduced using RT-PCR at positions 6752, 18997, 20460, and 21369, respectively. Especially nucleic acid sequences encoding the TGEV replicase, N protein, M protein, E protein or S protein of TGEV as used herein means nucleic acid sequences as disclosed in Penzes et al., 2001 (with or without the amendments mentioned above). It is of course also possible to use other TGEV strains or to include further deletions, substitutions, insertions or additions into the nucleic acid sequence. According to a further aspect the TGEV sequences thus differ from the sequences disclosed in Penzes et al. but still have a homology of at least 60%, preferably at least 75% and most preferably at least 95% to these sequences.

For the purposes of the present application sequence homology is determined using the clustal computer program available from the European Bioinformatics Institute (EBI), unless otherwise stated.

The nucleic acid of the present invention may further encode SEQ ID NO: 1 (gene 7 TRS inactivated plus the UTR 3'primer).

The infectious TGEV vector need not contain genes 3a, 3b and 7, as these are known to be non-essential. The proteins encoded by genes 3a, 3b and 7 of TGEV may modulate the immune response against TGEV and where it is desirable to modulate TGEV interaction with the host, these genes may be maintained in the TGEV vector.

The protein coding sequences within the nucleic acids of the present invention are preferably linked to sequences controlling the expression of these genes in the host cells or organisms. The genes encoding proteins capable of associating into VLPs may for example be flanked by transcription regulatory sequences (TRS) and/or internal ribosome entry site (IRES) sequences to increase transcription and/or translation of the protein coding sequences. Respective TRS and IRES sequences are well known in the art.

According to one aspect of the present invention, at least two of the viral proteins or fragments thereof encoded by the nucleic acid sequences of FMDV are expressed in the form of a polyprotein and the nucleic acid of the present invention further comprises sequences encoding a protease capable of digesting the polyprotein to obtain two or more separate proteins capable of associating into a virus-like particle that does not contain any infectious nucleic acid. This way of designing the expression constructs further increases the rate of protein association and thus the number of virus-like particles.

According to one aspect the FMDV virus-like particles thus produced are capable of generating an immune response in a mammal. The nucleic acids of the present invention may comprise sequences encoding FMDV proteins VP1, VP2, VP3, VP4 and/or 3C or sequences having a homology of at least 60%, preferably at least 75% and most preferably at least 95% to these sequences.

The FMDV P1 polyprotein (VP4, VP2, VP3 and VP1) is involved in the protection against FMDV. Three main neutralization sites have been described for FMDV serotype C: i) the mobile and protruding G-H loop of capsid protein VP1 termed antigenic site A, ii) the carboxi-terminal region of VP1, termed site C, and iii) the discontinuous site D involving residues of the carboxi-terminal region of VP1, the BC loop of VP2, and the BB knob of VP3. The 3C proteinase is responsible for processing P1. In a preferred embodiment the nucleic acids of the present invention may comprise sequences encoding FMDV polyprotein P1 and 3C.

The term "nucleic acids encoding FMDV proteins" is used herein to refer to nucleic acid sequences encoding genes of the FMDV clone C―S8cl as identified in the publication of Toja et al., 1999; the precise sequence of the FMDV genes is identified in Toja et al. by reference to an EMBL sequence (Accession No. AJ133357). According to the present invention the above term covers nucleic acid sequences having a homology of at least 60%, preferably at least 75% and most preferably at least 95% to the specific sequence identified. Corresponding nucleic acid sequenes can be obtained by passaging the virus (as shown in Toja) or by mutagenizing the viral genome, for example by substituting, deleting, inserting or adding nucleotides into the viral wild-type sequence.

The 3C gene of FMDV encodes a protease, which is capable of digesting a polyprotein obtained by expression of genes VP1-VP4. After digestion of the polyprotein into individual polypeptides, VLPs will be formed. The expression in the form of a polyprotein will increase the rate of VLP formation.

Additionally, the nucleic acids of the present invention may comprise sequences encoding FMDV protein 3D or sequences having a homology of at least 60%, preferably at least 75 and most preferably at least 95% to this sequence. The 3D gene of FMDV encodes the viral polymeras. The nucleotide sequence encoding the polymerase may encompass the full length sequence or a truncated sequence. It has been observed that for the purposes of generating an immune response a truncated version of the polymerase is sufficient. The truncated polymerase comprises at least consecutive 15 amino acids of the polymerase protein, perferably at least 30 or 50 consecutive amino acid residues of the full length protein. Specifically, the nucleotide sequence encoding the FMDV polymerase 3D may be truncated at the 5'end.

In accordance with the present invention the nucleic acid sequences encoding the polyprotein are preferably expressed under the control of the same regulatory sequences. According to an especially preferred embodiment the nucleic acid sequences encoding FMDV proteins VP1, VP2, VP3 and/or VP4 are expressed in the form of a polyprotein, optionally further comprising protein 3D of FMDV under the control of a strong promoter. Any strong promoter could be inserted, but it is preferred to use the natural promoter of the FMDV 3a gene, comprising the sequence:

GTTAATTCTATCATCTGCTATAATAGCAGTTGTTTCTGCTAGAGAATTTTGT-
TAAGGATGATGAATAAAGTCTTTAAGAACTAAACTTACGAGTCATTACAGGTCCTG
T (SEQ ID NO:2).

Independently or additionally the sequence encoding the FMDV protease 3C may be expressed under the control of a weak promoter, preferably under the control of the synthetic 22N promoter comprising the sequence:

AAAATTATTACATATGGTATAACTAAACAAA (SEQ ID NO:3).

The sequences encoding FMDV proteins may be derived from any FMDV isolate. However, preferably, sequences derived from FMDV serotypes O (isolate O panasia) or C (isolate C-Sta.pau.sp70) are used (Rowlands, 2003).

According to a further aspect of the present invention, the nucleic acids may be modified at specific positions. For example, the nucleic acids of the present invention may contain nucleotide sequences encoding FMDV serotype C capsid protein VP1 which is modified to obtain proteins with amino residues at positions 140-160 which differ from the natural residues in these positions. This approach broadens the immune response generated by vaccination using the nucleic acids.

The nucleic acids of the present invention may be in the form of DNA or RNA. Within the scope of the present invention specifically recombinant RNA molecules are encompassed which are encoded by one of the above nucleic acids.

According to a further aspect the present invention is directed towards vectors comprising one of the above nucleic acids. The vector can be a cDNA vector and preferably is a BAC derived vector, such as BAC-TGEV^{FL}. The vector is preferably capable of replicating the nucleic acid within a specific host cell or a number of host cells.

Host cells, which comprise a vector comprising one of the above nucleic acids are a further subject of the present invention. The cell may be a bacterial cell, an insect cell, a yeast cell, an animal cell, or a human cell. According to a preferred embodiment the cell is a porcine swine testis cell line, such as the cell line deposited under ATCC CRL1746.

A. further aspect of the present invention is directed to methods of preparing virus-like particles that do not contain any infectious nucleic acid which methods comprise steps, wherein a nucleic acid sequence as described above is expressed in a host cell in cell culture and the virus-like particles are isolated from the medium and/or from the host cells.

The present invention further is directed to pharmaceutical compositions comprising one of the nucleic acids, viral RNAs or vectors of the present invention or a host cell as described above. The pharmaceutical composition may further comprise a pharmaceutically acceptable carrier, excipient and/or adjuvants.

In a further embodiment the present invention relates to vaccines capable of protecting an animal against the disease caused by an infectious virus comprising a nucleic acid, a viral RNA, a vector or a host cell of the present invention. The vaccine may also comprise pharmaceutically acceptable carriers, excipients and/or adjuvants.

Adjuvants and carriers suitable for administering genetic vaccines and immunogens via the mucosal route are known in the art. Conventional carriers and adjuvants are for example reviewed in Kiyono et al 1996. The addition of chemokines that are used to modulate immune responses are also encompassed by the present invention. Respective compounds and their medical use has been reviewed in Toka et al. 2004. It is specifically advantagous to use one of granulocyte/macrophage colony-stimulating factor, interleukin-2 (IL-2), IL-12, IL-18. Combinatorial approaches utilizing several cytokines and chemokines might also be applied. In addition, as more is discovered regarding the requirements for memory development of T cells, boosters involving key cytokines such as IL-15 and IL-23 may prove beneficial to long-term maintenance of the memory pool.

The vaccine is preferably suitable for treating a mammal, for example a ruminant or a swine.

In accordance with the present invention vaccines are provided, which are preferably capable of inducing both a systemic immune response and a mucosal immune response against FMDV and/or TGEV.

The vaccine may be administered in accordance with methods routinely used in the art. Specifically vaccine may be administered by intramuscular, intravenous or oronasal administration.

One of the specific medical indications of the present invention is the use of the above vaccines for preventing the carrier state in an animal to be vaccinated.

The vaccines of the present invention allow one of ordinary skill to diagnose whether an animal is infected with a wild-type virus or has been vaccinated. According to a further aspect, the present invention is thus directed to methods for diagnosing whether an animal is infected with a virus or has been vaccinated using a vaccine of the present invention, which methods comprise steps, wherein the diagnosis uses antibodies specific for proteins of the wildtype virus not expressed by the vaccine strain. Differentiation of TGEV vaccinated animals from TGEV infected animals could alternatively be carried out using RT-PCR and sequence markers introduced into the recombinant TGEV genome at positions 6752, 18997, 20460, and 21369, which should encode G, C, T, and C, respectively.

The differentiation between vaccinated animals and wildtype FMDV, infected animals can be carried out using antibodies specific for proteins not present in the recombinant virus.

### Brief description of the Figures:

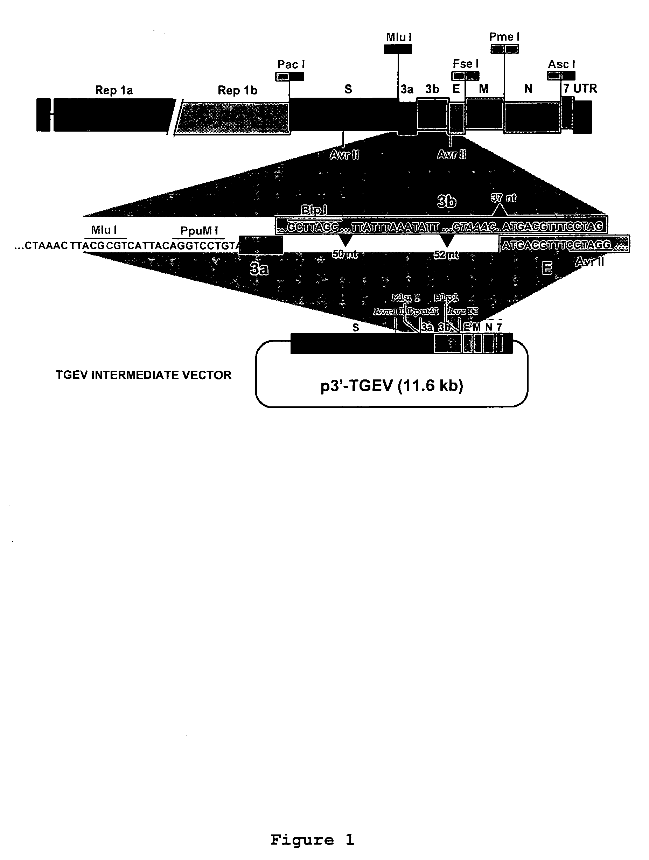

Figure 1 shows the genetic structure of TGEV intermediate vector p3'-TGEV. FMDV genes can be cloned in place of the TGEV genes 3a-3b, using the intermediate plasmid p3'-TGEV that contains the structural and non-structural TGEV genes located in the 3' third of the genome. The sequence of the 3a-3b region as well as the restriction sites that will be used for the cloning steps are indicated. The core sequence (CTAAAC) is indicated in italics. The viral genome is shown at the top. L, leader sequence; Rep 1a, rep 1b, S, 3a, 3b, E, M, N, an 7, TGEV genes; UTR, untranslated region.

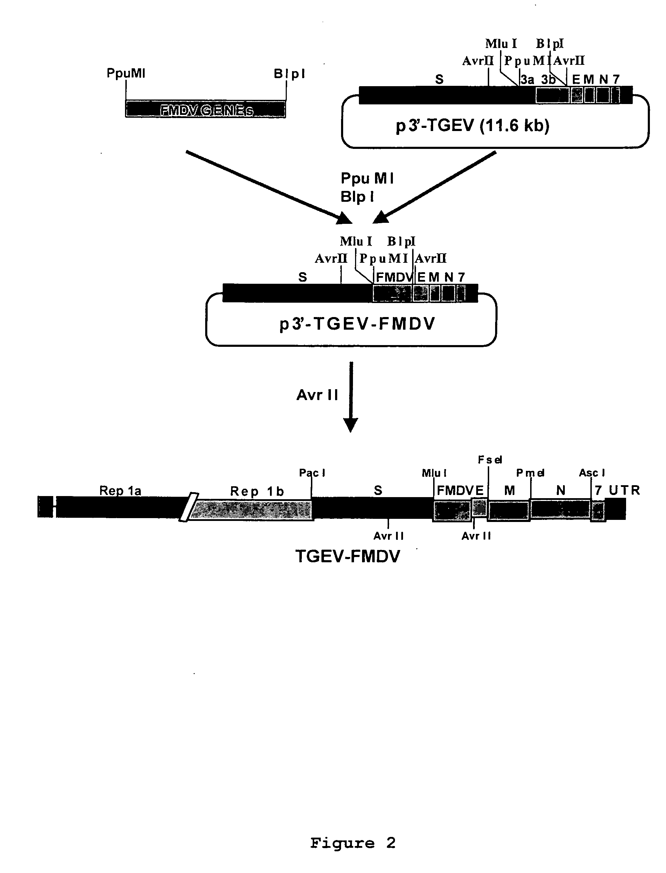

Figure 2 shows the strategy for the cloning of FMDV genes into TGEV vector. FMDV genes flanked by PpuMI and BlpI restriction sites were generated by PCR and cloned into the TGEV vector in two steps. PCR generated nucleic acids encoding FMDV antigens were cloned under the control of 3a TRS in p3'-TGEV digested with PpuMI and BlpI. Finally, recombinant vectors TGEV-FMDV were constructed by cloning the AvrII fragment from plasmids p3-TGEV-FMDV into the TGEV vector digested with AvrII.

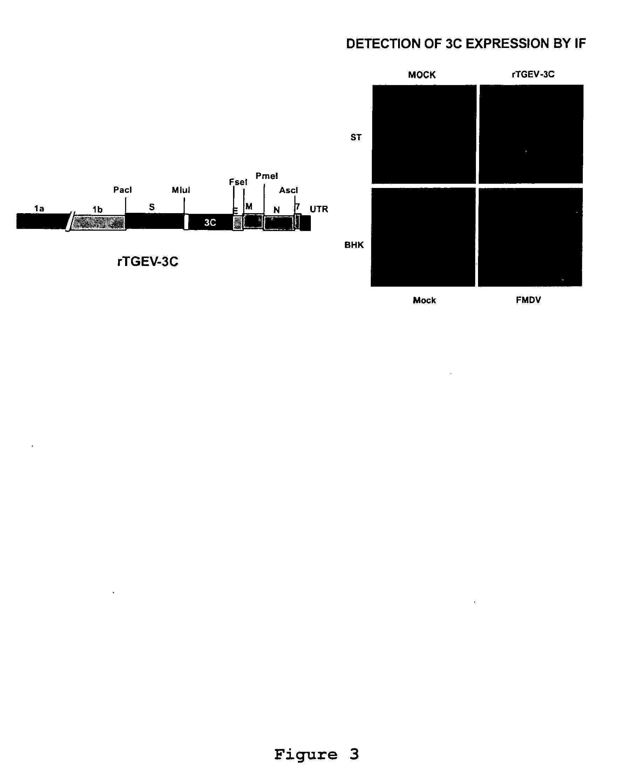

Figure 3 shows the analysis of an rTGEV derived vector expressing FMDV-3C. 3C of FMDV was cloned under the control of TRS3a in TGEV infectious cDNA clone, leading to the generation of the recombinant virus rTGEV-3C. Expression of FMDV-3C by the recombinant virus was detected at passage three by immunofluorescence in ST cells. As a positive control 3C expression in BHK cells infected with FMDV is indicated.

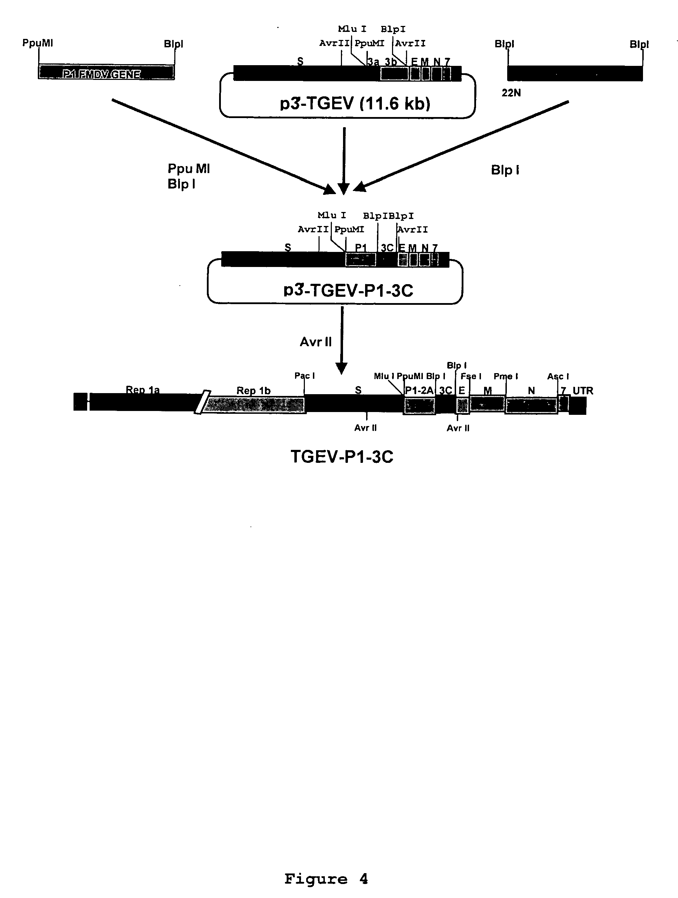

Figure 4 shows the generation of a dicistronic rTGEV derived vector expressing FMDV P1 and 3C genes. A dicistronic vector expressing FMDV genes P1 and 3C has been generated. In this construct the P1 and 3C genes were expressed under the control of the TGEV strong promoter of gene 3a and the synthetic 22N TGEV weak promoter, respectively. The strategy followed and the relevant restriction sites are indicated.

The following examples illustrate but do not limit the embodiments of the invention.

### EXAMPLE 1

### Growth of Eukaryotic Cells

TGEV growth, titration, and purification were performed in ST (swine testicle) cells, a cell line obtained from epithelial cells of fetal pig testicles (McClurkin and Norman, 1966). ST cells were obtained from L. Kemeny (National Animal Disease Centre, Ames, Iowa, USA).

Plasmid transfections assays were performed in Baby Hamster Kidney cells (BHK-21) stably transformed with the gene coding for the porcine aminopeptidase N (BHK-pAPN) (Laude et al., 1990). ST cells were cultivated in DMEM (Dulbecco's Modified Eagle Medium) supplemented with 10% fetal calf serum (FCS) (GIBCO-BRL), 50 mg/mL gentamicine, 2 mM glutamine, and 1% non-essential amino acids.

The BHK-21 stably transformed with the gene encoding for the porcine aminopeptidase N (BHK-pAPN) were grown in DMEM (Dulbecco's Modified Eagle Medium) supplemented with 2% fetal calf serum (FCS) (GIBCO-BRL), 50 mg/mL gentamicine, 2 mM glutamine, and 1% non-essential amino acids and Geneticine (G418) (1,5 mg/ml) as a selection agent.

### EXAMPLE 2

### Transformation of bacteria by plasmid electroporation

### Bacterial strains:

*Escherichia coli* DH10B (Gibco/BRL) (Hanahan et al., 1991) was the host for all the plasmids constructed. The genotype of this bacterial strain is: F⁻mcr A Δ (mrr-hsdRMS-mcrBC) φ80d*lac*ZΔM15 Δ*lac*X74 *deo*R *rec*A1 *end*A1 *ara*D139 (*ara,leu*) 7697 *gal*U *gal*K*_rsp*L *nup*G*.*

### Preparation of competent bacteria:

For amplification and production of electroporation-competent *E. coli* DH10B bacteria, the bacteria were grown in a SOB medium. Were inoculated 10 mL of SOB medium (20 g/L tryptone, 5 g/L yeast extract, 0,5 g/L NaCl) with a colony from a fresh plate, and were incubated for 12 h at 37°C under agitation. With 2 mL of this culture, 1 L of SOB medium supplemented was inoculated, and the culture was grown at 37°C to an optical density of 600 nm, between 0.8 and 0.9 absorbance units. Then the culture was cooled on ice for 20 min, and the bacteria were centrifuged in the Sorvall GSA rotor at 4,000 G for 15 min at 4°C. The bacteria were resuspended cold in 1 L of 10% glycerol. The bacteria suspension was centrifuged again and resuspended in 500 mL of 10% cold glycerol. The bacteria were sedimented and resuspended in 250 mL of 10% cold glycerol. Finally, the bacteria were centrifuged and resuspended in 3 mL of 10% glycerol. The final suspension was divided into aliquots parts of 50 µL and 100 µL and were kept at -70°C until they were used for electroporation. The transformation efficiency of the bacteria was calculated by electroporation with a known concentration of a pBluescript plasmid as a reference, and was reproducibly at about 10⁹ colonies/µg of DNA.

### Transformation of bacteria by plasmid electroporation:

50 µL of transformation-competent bacteria were mixed with 1 µL of each reaction mixture, or 10 ng of purified plasmid to the bacteria and incubated on ice for 1 min. Then, the mixture was transferred to 0.2 cm electroporation trays (Bio-Rad), and were transformed by a 2.5 kV electric pulse, 25 µF and 200 Ω in a "Gene Pulser" electroporator (Bio-Rad). After adding 1 mL of cold LB medium, the bacteria were incubated at 37°C under agitation for 1 h. Between 50 and 100 µL of the suspension of transformed bacteria were seeded in Petri dishes with LB (Luria-Bertani medium) in a solid medium (15 g/L agar) supplemented with ampicillin (100 µg/mL) or chloramphenicol (34 µg/mL). The bacteria grew for 16 h at 37°C (Bulllock, et al. 1987).

For production and purification of plasmids, the bacteria transformed with plasmids, that conferred ampicillin or chloramphenicol resistance, were grown from an isolated colony on a plate, in a liquid LB medium supplemented with 100 µg/mL of ampicillin or 34 µg/mL of chloramphenicol.

### EXAMPLE 3

### Plasmids for cloning of PCR products

The pGEM-T (Promega) plasmid was used to clone PCR products. This plasmid contains the T7 and SP6 bacteriophage promoters separated by the LacZ gene, interrupted by two protuberant T sequences between a multicloning sequences. This plasmid confers ampicillin resistance for its selection.

### EXAMPLE 4

### Manipulation of DNA

### Cloning and restriction enzymes:

For the manipulation and cloning of DNA, the restriction enzymes *Bam*HI, *Bbs* I, *Blp* I, *Eco* RI, *Mlu* I, *Swa* I, *Xcm* I, *Xho* I, were acquired from ROCHE or from New England Biolabs. Dephosphorylation of the DNA terminals, was done with shrimp alkaline phosphatase (SAP) (USB). A DNA ligation as T4 phage DNA ligase (New England Biolabs) was used. All the treatment with restriction enzymes, dephosphorylation, and DNA ligation were carried out by standard protocols previously described (Sambrook et al., 1989).

### Polymerase chain reaction (PCR):

To amplify DNA from a matrix, frequently plasmids, 50-100 ng of DNA plasmid was mixed with the corresponding oligonucleotides (10 µM), 0.25 mM deoxynucleotides triphosphate (ATP, GTP, TTP, and CTP), 1.25 mM MgCl₂, PCR buffer (10 mM Tris-HCl, pH 8.3, 50 mM KCl) and 2.5 U of Taq Gold DNA polymerase *(Thermus aquaticus)* (Roche), in a final volume of 50 µL. The reactions were carried out in the GeneAmp PCR System 9600 thermocycler from Perkin Elmer.

### Separation of DNA by agarose gel electrophoresis:

To separate DNA fragments, 1% agarose gels were used with ethydium bromide (1 µg/mL) in a 1X TAE buffer (40 mM Tris-acetate, 1mM EDTA).

### Purification of DNA:

The bacterial plasmids grown in the presence of the selection antibiotics were purified using the "Qiaprep Spin Miniprep kit" (Qiagen) to prepare of small quantities plasmid DNA, and the "Qiafilter Midi-Plasmid Kit" system (Qiagen) to prepare intermediate quantities of plasmid DNA. The DNA obtained from agarose gels was purified using the "QiaEx II Gel Extraction Kit" system (Qiagen). Purification of the PCR products was carried out by means of the system "QIA quick PCR Purification Kit" (Qiagen). In all cases, the manufacturer's instructions were followed.

### EXAMPLE 5

### RNA analysis

For analysis of the RNA produced in infections with TGEV clone PUR46-MAD, confluent monolayers of ST cells grown in 60 mm diameter culture plates (NUNC) were infected with viral inocula at a MOI of 1. The cells were lysed at 16 hpi [hours post infection] using a "RNeasy Mini Kit" (Qiagen), following the protocol provided by the commercial firm (Qiagen). The RNA was purified and resuspended in 40 µL of water treated with DEPC [diethyl polycarbonate] and 20 U of RNAse inhibitor (Roche).

### EXAMPLE 6

### Generation of rTGEV

The porcine transmissible gastroenteritis virus (TGEV) used belongs to the group of Purdue isolates, and was obtained in Indiana in 1946 (Doyle and Hutchings, 1946). The virus was adapted to grow in cell cultures (Haelterman and Pensaert, 1967), and was provided by E. H. Bohl (Ohio State University, Wooster Ohio). This TGEV isolate has been passaged in ST cells 115 times, and has been cloned five times consecutively in Dr. Luis Enjuanes laboratory (Centro Nacional de Biotecnologia, Madrid, Spain). The clone selected was labelled PUR46-CC120-MAD, abbreviated PUR46-MAD. This is an attenuated virus that grows well in cell cultures, and reaches titers between 10⁸ and 10⁹ PFU/mL.

rTGEV viruses were generated from pBAC-TGEV constructs containing the S gene from the virulent TGEV strain PUR-C11 (S_{c11}) as described (Alamazan et al., 2000 ; Gonzalez et al., 2002). Viruses containing the S gene (encoding the TGEV spike protein) from the attenuated strain PTV (S_{PTV}) were derived from the corresponding pBAC-TGEV vectors with S_{c11} by replacing this gene by the S_{PTV} of the respiratory strain.

### EXAMPLE 7

### Construction of rTGEV vectors expressing FMDV proteins

In one approach the FMDV antigens 3C, the 5' P1 (encoding VP4, VP2 and the 5' end of VP3), and the 3' P1 (encoding VP3 and VP1) were expressed in the BAC-TGEV^{FL} . The nucleic acids encoding FMDV antigens were generated by PCR using specific oligonucleotides including restriction sites PpuMI and BlpI for direct cloning of the amplicons in the TGEV intermediate vector p3'-TGEV (Fig. 1). This intermediate vector contained the structural and non-structural TGEV genes, including genes 3a and 3b, located at the 3' third of the genome. PCR generated nucleic acids encoding FMDV antigens were digested with PpuMI and BlpI and cloned into p3'-TGEV in place of TGEV genes 3a and 3b under the control of 3a natural transcription regulating sequence (TRS). From the intermediate plasmid generated, a AvrII fragment containing the nucleic acid encoding the FMDV antigens under the control of 3a TRS was cloned in the TGEV full-length cDNA clone (BAC-TGEV^{FL}) to generate the recombinant vectors rTGEV-C, rTGEV-5'P1 and rTGEV-3'P1 (Fig 2.).

In another approach a dicistronic vector expressing FMDV antigens P1 and 3C was generated. This vector allows the expression of both antigens in the same cell and the generation of VLPs after processing of polyprotein P1 by proteinase 3C. P1 and 3C were expressed under the control of TGEV strong promoter of gene 3a and the 22N TGEV weak promoter (this promoter includes 22 nt of the 5'TRS of gene N, the conserved CS sequence, and the 3'TRS of gene M), respectively. FMDV gene P1, flanked by PpuMI and BlpI restriction sites, and 3C by BlpI restriction site, were generated by PCR and cloned in two steps into p3'-TGEV to generate p3'-TGEV-P1-3C. In a first step, P1 digested with PpuMI and BlpI was cloned into p3'-TGEV digested with the same enzymes to generate p3'-TGEV-P1. Secondly, p3'-TGEV-P1-3C was generated by cloning of 3C digested with BlpI into p3'-TGEV-P1. Finally, the recombinant vector TGEV-P1-3C was constructed by cloning the AvrII fragment from plasmid p3'-TGEV-P1-3C (Fig. 4). The recombinant virus is being rescued. The stability, transcription and expression efficiency of recombinant TGEV derived virus expressing FMDV antigens P1 and 3C can be analyzed in cell culture. Additionally, natural TRSs, modified TRSs, IRES or a combination of them can be used to optimize the expression of FMDV antigens.

### EXAMPLE 8

### Analysis of recombinant TGEV derived viruses expressing FMDV proteins

The stability, transcription and expression efficiencies of recombinant TGEV derived viruses expressing FMDV antigens can be analyzed in cell culture.

BHK-pAPN cells (BHK cells expressing the cellular aminopeptidase N receptor) were transfected with the recombinant vector expressing FMDV 3C or 5' P1 and the recombinant viruses were rescued. Recombinant viruses rTGEV-C and rTGEV-5' P1 were stable in cell cultures and expressed high amount of 3C and 5' P1. The recombinant virus showed a plaque morphology similar to that of the control virus (rTGEV). The stability and expression level of the protein was analysed by immunofluorescence. As an example the results for FMDV 3C are shown in Figure 3.

### Bibliography

Alamazan, F., Gonzales J.M., Penzes Z., Izeta A., Calvo E., Plana-Duran J., and Luis Enjuanes (2000). Proc. Natl. Acad. Sci. USA **97:** 5516-5521.
Alonso, S., Izeta A., Sola I., and Enjuanes L. (2002a). Transcription regulatory sequences and mRNA expression levels in the coronavirus transmissible gastroenteritis virus. J. Virol. **76**:1293-1308
Alonso, S., Sola, I., Teifke, J., Reimann, I., Izeta, A., Balach, M., Plana-Durán, J., Moormann, R. J. M., and Enjuanes, L. (2002b). In vitro and in vivo expression of foreign genes by transmissible gastroenteritis coronavirus-derived minigenomes. *J*. *Gen. Virol.* **83**, 567-579.
Bullock, W., Fernández, J.M. and Short, J.M. (1987). XL1-Blue: a high efficiency plasmid transforming recA *E.coli* strain with P-galactosidadse selection. Biotechniques **8**:26-27.
Chang, K.-Y., and Tinoco, I. (1994). Characterization of a "kissing" hairpin complex derived from the human immunodeficiency virus genome. *Proc. Natl. Acad. Sci. USA* **91**, 8705-8709.
Delmas B, Gelfi J., Kut E., Sjostrom H., Noren O. , Laude H. (1994). Determinants essential for the transmissible gastroenteritis virus-receptor interaction reside within a domain of aminopeptidase-N that is distinct from the enzymatic site. J Virol. **68(8)**:5216-24.
Doyle, L.P. and Hutchings, L.M. (1946). A transmissible gastroenteritis in pigs. J. Amer. Vet. Med. Assoc. **108**:257-259.
Enjuanes L., et al. (2003). Virus based vectors for gene expression in mammalian cells; Coronaviruses; in *Gene transfer and expression in mammalian cells;* Ed. S.C. Makrides, p. 151-158.
Gonzalez, J.M., Penzes Z., Almazan F., Calvo E., and Luis Enjuanes. (2002) Stabilization of a full-length infectious cDNA clone of transmissible gastroenteritis coronavirus by the insertion of an intron. J. Virol. **76**: 4655-4661.
Haelterman E.O. and Pensaert M.B. (1967). Pathogenesis of transmissible gastroenteritis of swine. Proc. 18th World Vet. Congress **2**:569-572.
Hanahan D., Jessee J., Bloom F.R. (1991). Plasmid transformation of Escherichia coli and other bacteria. Methods Enzymol. **204**: 63-113.
Izeta, A., Smerdou, C., Alonso, S., Penzes, Z., Méndez, A., Plana-Durán, J., and Enjuanes, L. (1999). Replication and packaging of transmissible gastroenteritis coronavirus-derived synthetic minigenomes. *J. Virol.* **73**, 1535-1545.
Kim, Y., Kyeong-Ok, Kim W., Saif L. J. (2002). Production of Hybrid Double or Triple-Layered Virus Like Particles of Group A and C Rotaviruses using a Baculovirus expression system. Virology **302**: 1-8.
Kiyono et al. (1996). Mucosal Vaccines. Academic Press, New York.
Laude, H., Rasschaert D., Delmas B., Godet M., Gelfi J., and Bernard C. (1990). Molecular biology of transmissible gastroenteritis virus. Vet. Microbiol. **23**: 147-154.
McClurkin, A.W. and Noman, J.O. (1966). Studies on transmissible gastroenteritis of swine. II. Selected characteristics of a cytopathogenic virus common to five isolates from transmissible gastroenteritis. Can. J. Comp. Med. Vet. Sci. **30**:190-198.
Ortego J., Escors D., Laude H., and Luis Enjuanes (2002). Generation of a replication-competent, propagation deficient virus vector based on the transmissible gastroenteritis coronavirus genome. J. Virol. **76**:11518-11529.
Ortego J., Sola I., Almazan F., Ceriani J.E., Riquelme C., Balach M., Plana-Duran J. and Luis Enjuanes (2003). Transmissible gastroenteritis coronavirus gene 7 is not essential but influences *in vivo* replication and virulence. Virology **308**: 13-22.
Penzes Z., Gonzales J.M., Calvo E., Izeta A., Smerdou C., Mendez A., Sanches C.M., Sola I., Almazan F., Enjuanes L. (2001). Complete genome sequence of Transmissible Gastroenteritis Coronavirus PUR46-MAD clone and evolution of the purdue virus cluster. Virus Genes **23**:1, 105-118.
Rowlands, D.J. Editor (2003). Foot-and-mouth disease. Virus Research **91**: 1- 161.
Sambrook et al. (1989). Molecular Cloning: A Laboratory Manual. Ed Cold Spring Harbor Laboratory.
Sánchez, C. M., Izeta, A., Sánchez-Morgado, J. M., Alonso, S., Sola, I., Balasch, M., Plana-Durán, J. and Enjuanes, L. (1999). Targeted recombination demonstrates that the spike gene of transmissible gastroenteritis coronavirus is a determinant of its enteric tropism and virulence. J. Virol. **73**:7607-7618.
Sawicki & Sawicki (1990) Coronavirus transcription: subgenomic mouse hepatitis virus replicative intermediates function in RNA synthesis. J Virol. **64**(3):1050-6.
Schwartz-Cornil I., Benureau Y., Greenberg H., Hendrickson B.A., Cohen J. (2002) Heterologous protection induces by the inner capsid proteins of rotavirus requires transcytosis of mucosal immunoglobulins. J. Virol. **76**: 8110-8117.
Sethna, P. B., Hung, S.-L., and Brian, D. A. (1989). Coronavirus subgenomic minus-strand RNAs and the potential for mRNA replicons. *Proc. Natl. Acad. Sci. USA* **86,** 5626-5630.
Siddell, S. G. (1995). "The *Coronaviridae."* The Viruses (H. Fraenkel-Conrat, and R. R. Wagner, Eds.) Plenum Press, New York.
Sola I, Alonso S., Zúñiga S., Balasch M., Plana-Duran J., Enjuanes L. (2003) Engineering the transmisible gastroenteritis virus genome as an expression vector inducing lactogenic immunity. J. Virol. **77**:4357-4369.
Toja M., Escarnis C., Domingo E. (1999). Genomic nucleotide sequence of a foot-and-mouth disease virus clone and its persistent derivatives - Implications for the evolution of viral quasispecies during a persistent infection. Virus Research **64**:161-171.
Toka, F.N. et al. (2004). Molecular adjuvants for mucosal immunity. Immunol Rev. **199**:100-112.
van der Most, R. G., and Spaan, W. J. M. (1995). Coronavirus replication, transcription, and RNA recombination. *In* "The Coronaviridae" (S. G. Siddell, Ed.), pp. 11-31. Plenum Press, NewYork.

## Claims

1. Nucleic acid comprising:
(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and
(b) sequences encoding one or more proteins of foot and mouth disease virus (FMDV), wherein the one or more proteins are capable of associating into a virus-like particle (VLP) that does not contain any infectious nucleic acid.

2. Nucleic acid according to claim 1, wherein the nucleic acid encodes a TGEV replicase and a sequence encoding the TGEV N protein.

3. Nucleic acid according to claim 1 or 2, wherein the nucleic acid further encodes SEQ ID NO:1 or a sequence having a homology of at least 60% to SEQ ID NO.:1.

4. Nucleic acid according to any one of claims 1 to 3, wherein the replication competent TGEV vector is not infectious.

5. Nucleic acid according to any one of claims 1 to 3, wherein the replication competent TGEV vector is infectious.

6. Nucleic acid according to claim 5, wherein the nucleic acid further comprises one or more of the following TGEV genes: S, E, M and/or N or sequences having a homology of at least 60% to the given sequence.

7. Nucleic acid according to claim 5 or 6, wherein the TGEV infectious viral particles obtainable from the association of TGEV proteins and the nucleic acid sequences are attenuated viral particles.

8. Nucleic acid according to one of claims 1 to 7, wherein at least two of the viral proteins encoded by the nucleic acid sequences **characterized in** (b) of claim 1 are expressed in the form of a polyprotein and the nucleic acid further comprises sequences encoding a protease capable of digesting the polyprotein to obtain two or more separate proteins capable of associating into a virus like particle (VLP) that does not contain any infectious nucleic acid.

9. Nucleic acid according to one of claims 1 to 8, wherein the virus like particles of FMDV are capable of generating an immune response in a mammal.

10. Nucleic acid according to any of claims 1 to 9, wherein the nucleic acid sequences encoding FMDV proteins comprise sequences encoding at least two of the following proteins:
VP1, VP2, VP3, VP4 or 3C;
or sequences having a homology of at least 60% to these sequences.

11. Nucleic acid according to any of claims 1 to 10, further comprising sequences encoding FMDV protein 3D or sequences having a homology of at least 60% to this sequence.

12. Nucleic acid according to one of claims 1 to 11, wherein the nucleotide sequence encoding the FMDV polymerase 3D gene is truncated.

13. Nucleic acid according to one of claims 1 to 12, wherein the nucleotide sequence encoding the FMDV polymerase 3D is truncated at the 5' end.

14. Nucleic acid according to any of claims 1 to 9, wherein the nucleic acid sequences encoding FMDV proteins comprise sequences encoding the FMDV polyprotein P1 (VP4, VP2, VP3, and VP1) and the 3C protein, or sequences having a homology of at least 60% to these sequences.

15. Nucleic acid according to one of claims 1 to 14, wherein the nucleic acid sequences encoding FMDV proteins VP1, VP2, VP3 and VP4 is expressed in the form of a polyprotein, which polyprotein optionally further comprises protein 3D.

16. Nucleic acid according to claim 15, wherein proteins VP1, VP2, VP3 and VP4 and optionally protein 3D are expressed in the form of a polyprotein under the control of a strong promoter, preferably a promoter comprising the natural promoter of the FMDV 3a gene, which comprises SEQ ID NO:2.

17. Nucleic acid according to one of claims 1 to 16, wherein the sequence encoding FMDV protease 3C is expressed under the control of a weak promoter, preferably under the control of the synthetic 22N promoter comprising SEQ ID NO:3.

18. Nucleic acid according to any of claims 1 to 17, wherein the sequences encoding FMDV proteins are sequences derived from FMDV serotypes O (isolate O PanAsia) or C (isolate C-Sta.Pau/Sp70).

19. Nucleic acid according to any of claims 1 to 18, wherein the nucleotide sequence encoding FMDV serotype C capsid protein VP1 is modified to obtain proteins with modified amino acid residues at position 140 to 160.

20. Nucleic acid according to any of claims 1 to 19, which is DNA or RNA.

21. Recombinant RNA encoded by a nucleic acid according to any of claims 1 to 20.

22. Vector comprising a nucleic acid according to one of claims 1 to 21.

23. Vector according to claim 22, wherein the vector is a cDNA vector.

24. Vector according to claim 23, wherein the vector is a BAC-TGEV^{FL} vector.

25. Vector according to any of claims 22 to 24, wherein the vector is capable of replicating the nucleic acid within a host cell.

26. Host cell comprising a vector according to one of claims 22 to 25.

27. Host cell according to claim 26, wherein the cell is a bacterial cell, an insect cell, a yeast cell, an animal cell or an isolated human cell.

28. Host cell according to claim 27, wherein the cell is a porcine swine testis cell line, such as the cell line deposited under ATCC CRL-1746.

29. Virus particle comprising a nucleic acid according to any of claims 1 to 20 and at least one TGEV coat protein.

30. Virus particle according to claim 29, comprising all TGEV coat proteins of the native TGEV virus particle.

31. Method of preparing virus like particles that do not contain any infectious nucleic acid, comprising steps, wherein a nucleic acid sequence according to any of claims 1-20 is expressed in a host cell in cell culture and the virus like particles are isolated from the medium and/or from the host cells.

32. Pharmaceutical preparation comprising a nucleic acid according to one of claims 1 to 20, a viral RNA or vector according to one of claims 21 to 25, a host cell according to one of claims 26 to 28 or a virus particle according to claim 29 or 30.

33. Pharmaceutical preparation according to claim 32 further comprising a pharmaceutically acceptable carrier, excipient and/or adjuvants.

34. Vaccine capable of protecting an animal against a disease caused by an infectious virus comprising a nucleic acid according to one of claims 1 to 20, a viral RNA or vector according to one of claims 21 to 25 , a host cell according to one of claims 26 to 28 or a virus particle according to claim 29 or 30.

35. Vaccine according to claim 34 further comprising a pharmaceutically acceptable carrier, excipient and/or adjuvants.

36. Vaccine according to claim 34 or 35, wherein the vaccine is suitable for vaccinating a ruminant or a swine.

37. Vaccine capable of protecting an animal against foot and mouth disease comprising a virus particle, wherein the virus particle comprises a nucleic acid according to claim 10 or 11 and at least one or all of the TGEV coat proteins.

38. Vaccine according to any of claims 34 to 37, wherein the vaccine is capable of inducing both a systemic immune response and a mucosal immune response against infectious viral agents.

39. Vaccine according to any of claims 34 to 38, wherein the infectious agent is FMDV or TGEV.

40. Use of a vaccine according to any of claims 34 to 39 for the protection of animals against viral infection, wherein the vaccine is administered by intramuscular, intravenous or oronasal administration.

41. Use of a vaccine according to claim 40 for preventing the carrier state in said animals.

42. Method for diagnosing whether an animal is infected with a virus or has been vaccinated using a vaccine according to one of claims 34 to 39, comprising steps, wherein the diagnosis uses antibodies specific for proteins of the wild type virus but not expressed by the vaccine strain.


SEQUENCE LISTING

<110> Consejo Superior De Investigaciones Cientificas

<120> Nucleic Acid Sequence encoding FMDV Proteins capable of associating
into a Virus-like Particle

<130> P 62269

<160> 3

<170> PatentIn version 3.1

<210> 1

<211> 534

<212> DNA

<213> Artificial Sequence

<220>

<223> Pos. 1-24: TGEV gene 7 TRS inactivated; Pos. 25-261: TGEV gene 7;
Pos. 262-534 3'UTR

<400> 1
tgatgaggta acgaactaaa cgagatgctc gtcttcctcc atgctgtatt tattacagtt 60

ttaatcttac tactaattgg tagactccaa ttattagaaa gactattact taatcactct 120

ttcaatctta aaactgtcaa tgactttaat atcttatata ggagtttagc agaaaccaga 180

ttactaaaag tggtgcttcg agtaatcttt ctagtcttac taggattttg ctgctacaga 240

ttgttagtca cattaatgta aggcaacccg atgtctaaaa ctggtttttc cgaggaatta 300

ctggtcatcg cgctgtctac tcttgtacag aatggtaagc acgtgtaata ggaggtacaa 360

gcaaccctat tgcatattag gaagtttaga tttgatttgg caatgctaga tttagtaatt 420

tagagaagtt taaagatccg ctacgacgag ccaacaatgg aagagctaac gtctggatct 480

agtgattgtt taaaatgtaa aattgtttga aaattttcct tttgatagtg atac 534

<210> 2

<211> 109

<212> DNA

<213> Foot-and-mouth disease virus

<400> 2
gttaattcta tcatctgcta taatagcagt tgtttctgct agagaatttt gttaaggatg 60

atgaataaag tctttaagaa ctaaacttac gagtcattac aggtcctgt 109

<210> 3

<211> 31

<212>  DNA

<213>  Artificial Sequence

<220>

<223>  Synthetic promotor sequence

<400>  3
aaaattatta catatggtat aactaaacaa a                                    31